# EUROPEAN PATENT SPECIFICATION

(11) **EP 3 930 775 B1**
(45) Date of publication and mention of the grant of the patent: **11.10.2023**
(21) Application number: 20714071.6
(22) Date of filing: 27.02.2020
(51) Int. Cl.: A61L 27/18, A61L 27/34, A61L 29/06, A61L 29/08, A61L 29/18, A61L 31/06, A61L 31/10, A61L 31/18

(54) **RADIOPAQUE MEDICAL COMPONENTS AND DEVICES**
RÖNTGENOPAKE MEDIZINISCHE KOMPONENTEN UND VORRICHTUNGEN
COMPOSANTS ET DISPOSITIFS MÉDICAUX RADIO-OPAQUES

(30) Priority: 01.03.2019 US 201962812468 P; 12.04.2019 US 201962833102 P; 03.05.2019 EP 19172531
(43) Date of publication of application: 05.01.2022
(73) Proprietor: DSM IP Assets B.V., 6411 TE Heerlen (NL)
(72) Inventor: DE BONT, Nicolaes Hubertus Maria, 6100 AA Echt (NL); LEBOUILLE, Jérôme George Jozeph Louis, 6100 AA Echt (NL); DAVISON, Noel L., Columbia, Maryland 21045 (US)
(74) Representative: DSM Intellectual Property
(86) International application number: PCT/US2020/020132
(87) International publication number: WO 2020/180613

(56) References cited:
- WO-A1-2005/065725
- US-A1- 2006 058 867

## Description

### Field

The disclosed inventions pertain to materials, methods, and devices that may be radiopaque and may be useful in the medical field.

### Background

Radiopacity in the medical field is the quality of inhibiting x-rays or similar radiation used in medical imaging. Medical devices often contain a radiopacifier to enhance visualization during various medical procedures. Examples of devices that may contain radiopacifiers are catheters, guidewires, stents, and implanted medical devices. Including a radiopacifier in such a device acts as a visual identifier enabling a physician to accurately place a device or monitor the position of a permanently implanted device.

Examples of radiopacifiers are platinum, titanium, tungsten, barium sulfate, and zirconium oxide.

A radiopacifier may be incorporated into a medical device in a number of different ways. In metal devices, radiopaque materials, such as platinum, may be crimped or soldered onto the device to add radiopacity. In polymer devices, particles of a radiopacifier may be blended with the polymer prior to injection molding the medical device.

Examples of documents discussing imparting radiopacity to polymer articles are: US5300048, US8334524, US20060058867, US20080221670, US20110305881, WO2005/030284, WO2005/065725, and WO2006/132850.

### Summary

There are specific challenges in developing radiopaque polymers. First, the use of a radiopacifier will increase the viscosity of the polymer formulation. A higher viscosity of the polymer formulation may negatively impact the ease of forming a radiopaque component or coating. Second, the addition of a radiopacifier may impact the final mechanical properties of the formed component or coating. The required mechanical properties of the radiopaque component or coating will depend on the desired function of the medical device, and thus whether the resulting mechanical properties of the radiopaque polymer component or coating is sufficient will depend on the intended application of the medical device. Third, the radiopacity should be appropriately tuned. Depending on the given use of the device, the radiopaque portion may need to contrast with other elements of the device to allow for proper positioning or visualization of the device.

The most common way of forming a radiopaque polymer is through compounding the radiopacifier and base polymer, then applying heat-based component manufacturing methods, such as extrusion or injection molding, to produce a component. However, these types of materials are generally hard and brittle, owing to the hard nature of the radiopacifier itself, which are usually dense metals, and the inelastic (stiff) nature of the polymer. Although heat-process manufacturing methods are the most obvious method of choice, they may also introduce polymer degradation especially in the presence of high amounts of hygroscopic radiopacifier material (e.g., barium sulfate, bismuth trioxide, etc.). The hard, brittle nature of compounded, heat-processed radiopaque polymers makes them unsuitable for many medical implant applications that require compliance to native tissue or high crimpability and elasticity during delivery and function.

Radiopaque polymer components may also not be suitable in certain circumstances because they may be too stiff and impact the flexibility or maneuverability of the medical device. Such flexibility and maneuverability may be important for the percutaneous delivery of low profile medical devices such as cardiovascular devices. For instance, certain flexibility and compressibility of a device may be necessary to crimp it to the desired profile to deliver it to the target anatomical location in catheter-based procedures. Furthermore, radiopaque polymer components may be secured to a medical device by melting, heat shrinking, or otherwise bonding to the surface of the medical devices. Such heat-based processes can introduce thermal stress to the device component and decrease its fatigue lifetime and increase the chances of stress cracking under load.

Radiopaque polymer coatings or adhesives have also been disclosed in the prior art. Coatings or adhesives may be preferred because they can be formed to the specific geometry of the medical device and without substantial changes to the dimensions of the device. However, for internal use, polymer coatings or adhesives present an increased possibility of a portion of the coating being separated from the medical device in the body. Such debonding of the coating can result in clinical complications such as embolism or stroke, depending on the location and nature of the medical device containing the coating. Generally, this possibility increases for devices where the device will undergo substantial motion or where the device must be flexible under cyclic loading.

The inventors sought to overcome these challenges by providing a radiopaque material in a composition and form that may present improvements over the prior art in mechanical properties, suture retention strength, softness, Young's modulus, ultimate strength, elongation at break, viscosity, processability, usability, assembly, convenience, flexibility, crimpability, coefficient of friction, compliance to native tissue, visualization, and/or durability.

In an embodiment, a medical component comprises from 5 to 50 wt% of a polyurethane and from 50 to 95 wt% of a radiopacifier, based on the total weight of the medical component, wherein the medical component has a thickness of from 0.025 to 1 mm, and wherein the polyurethane comprises a backbone that comprises the reaction product of a diisocyanate, a polymeric aliphatic diol, and a chain extender, and wherein the polymeric aliphatic diol comprises a polysiloxane diol and a polycarbonate diol. A medical component is a component of a medical device. For example, a medical component may be attached or otherwise incorporated into a medical device

In an embodiment, the medical component has a length of from 5 to 100 mm and a width of from 1 to 6 mm. In an embodiment, the medical component has a suture retention strength of from 3 to 9 N. In an embodiment, a medical device comprising radiopacity comprises the medical component sutured to the medical device.

In an embodiment, a composition comprises from 5 to 50 wt% of a polyurethane, wherein the polyurethane comprises a backbone that comprises the reaction product of a diisocyanate, a polymeric aliphatic diol, and a chain extender, and wherein the polymeric aliphatic diol comprises a polysiloxane diol and a polycarbonate diol, and from 50 to 95 wt% of a radiopacifier, based on the total dry weight of the composition, and from 80 to 99 wt% solvent, based on the total weight of the composition. The compositions may be useful for forming medical components or coatings.

The thickness of the medical components of the invention are not typically attainable by injection molding or extrusion processes. In an embodiment, a method of forming a medical component comprises the steps of:
a. forming a composition comprising from 5 to 50 wt% of a polyurethane, wherein the polyurethane comprises a backbone that comprises the reaction product of a diisocyanate, a polymeric aliphatic diol, and a chain extender, and wherein the polymeric aliphatic diol comprises a polysiloxane diol and a polycarbonate diol, and from 50 to 95 wt% of a radiopacifier, based on the total dry weight of the composition, and a solvent,
b. casting the composition into a film,
c. evaporating the solvent, thereby obtaining the medical component,
wherein the medical component has a thickness of from 0.025 to 1 mm.

In an embodiment, a method of forming a medical device comprising radiopacity comprises the steps of:
a. providing a medical component comprising from 5 to 50 wt% of a polyurethane, wherein the polyurethane comprises a backbone that comprises the reaction product of a diisocyanate, a polymeric aliphatic diol, and a chain extender, and wherein the polymeric aliphatic diol comprises a polysiloxane diol and a polycarbonate diol, and from 50 to 95 wt% of a radiopacifier, based on the total weight of the medical component, wherein the medical component has a thickness of from 0.025 to 1 mm,
b. attaching the medical component to a medical device.

The compositions, medical components, medical devices, and/or methods, disclosed herein may exhibit benefits in film formation, reproducibility, mechanical properties, such as modulus, tensile strength, elongation, durability, or tear strength, suture retention strength, isotropy of mechanical properties, anti-fouling, viscosity of compositions that may be used to form the medical components, process reproducibility, process speed, usability, assembly, convenience, visualization, durability, surface quality, use with a wide range of solvents, and/or health and safety concerns, such as easier or more expedient removal of residual solvent.

### Brief Description of the Drawings

Fig. 1 is a graph of Ultimate Tensile Strength (MPa) associated with Example 2.
Fig. 2 is a graph of Elongation at Break (%) associated with Example 2.
Fig. 3 is an image obtained from a fluoroscope associated with Example 3.

### Detailed Description

In accordance with an embodiment, a medical component comprises from 5 to 50 wt% of a polyurethane and from 50 to 95 wt% of a radiopacifier, based on the total weight of the medical component, wherein the medical component has a thickness of from 0.025 to 1 mm, and wherein the polyurethane comprises a backbone that comprises the reaction product of a diisocyanate, a polymeric aliphatic diol, and a chain extender, and wherein the polymeric aliphatic diol comprises a polysiloxane diol and a polycarbonate diol. A medical device may comprise one or more medical components. A radiopaque medical component according to the invention is formed from a composition comprising a polyurethane and a radiopacifier.
The polyurethane is formed from a formulation.

### Polyurethane

The polyurethane comprises a backbone that comprises the reaction product of a diisocyanate, a polymeric aliphatic diol, and a chain extender. In an embodiment, the polyurethane consists of a backbone that comprises the reaction product of a diisocyanate, a polymeric aliphatic diol, and, a chain extender. In an embodiment, the polyurethane further comprises an endgroup. In an embodiment, the polyurethane is thermoplastic. In an embodiment, the polyurethane is thermoset. In an embodiment, the polyurethane is linear. In an embodiment, the polyurethane is branched.

By a reaction product it is meant that the diisocyanate and polymeric aliphatic diol, and the chain extender, are engaged in a simultaneous or sequential chemical reaction For example, a reaction product of a diisocyanate, a polymeric aliphatic diol, and a chain extender is formed i) when the diisocyanate, polymeric aliphatic diol, and chain extender are all reacted together simultaneously, or ii) when a pre-polymer is first formed by reacting the diisocyanate and the polymeric aliphatic diol, and then this prepolymer is subsequently reacted with the chain extender.

In an embodiment, the polyurethane is devoid of a hydrophilic polymer moiety. Examples of hydrophilic polymer moieties are polyethylene oxide or polyoxazoline moieties.

In an embodiment, the polyurethane has a number average molecular weight (Mn) of at least 10,000 g/mol, 50,000 g/mol, 100,000 g/mol, 150,000 g/mol, 200,000 g/mol, or 250,000 g/mol. In an embodiment, the polyurethane has a Mn of 1,000,000 g/mol or less, 800,000 g/mol or less, 700,000 g/mol or less, 600,000 g/mol or less, or 500,000 g/mol or less.

In an embodiment the polyurethane is present in the medical component in an amount of 5 wt% or more, 6 wt% or more, 8 wt% or more, 10 wt% or more, 12 wt% or more, 15 wt% or more, 20 wt% or more, or 25 wt% or more, based on the total weight of the medical component. In an embodiment the polyurethane is present in the medical component in an amount of 50 wt% or less, 45 wt% or less, 40 wt% or less, 35 wt% or less, 30 wt% or less, 25 wt% or less, 20 wt% or less, 15 wt% or less, or 10 wt% or less, based on the total weight of the medical component.

In an embodiment the polyurethane is present in the composition in an amount of 8 wt% or more, based on the total dry weight of the composition. By dry weight it is meant the total weight of the composition excluding any solvents. In an embodiment the polyurethane is present in the composition in an amount of 20 wt% or less, 15 wt% or less, 12 wt% or less, 10 wt% or less, 8 wt% or less, 7 wt% or less, or 6 wt% or less, based on the total dry weight of the composition.

Various required and optional components of the polyurethane are described in further detail in the following sections.

### Diisocyanate

The backbone of the polyurethane comprises the residue of a diisocyanate. In an embodiment, the diisocyanate comprises an average of at least 1.9 isocyanate groups per molecule and an average of less than 2.7 isocyanate groups per molecule.

In an embodiment, the diisocyanate comprises an aliphatic diisocyanate. In an embodiment, the diisocyanate comprises an aromatic diisocyanate. In an embodiment, the diisocyanate comprises 4,4'-diphenylmethane diisocyanate (MDI), 2,4-toluene diisocyanate, 2,6-toluene diisocyanate, 1,4-phenylene diisocyanate, hexamethylene diisocyanate (HDI), tetramethylene-1,4-diisocyanate, cyclohexane-1,4-diisocyanate, dicyclohexylmethane-4,4'-diisocyanate (HMDI), isophorone diisocyanate (IPDI), or a mixture thereof. In an embodiment, the diisocyanate comprises hexamethylene diisocyanate, dicyclohexylmethane 4,4'-diisocyanate, isophorone diisocyanate, or a mixture thereof. In an embodiment, the diisocyanate consists of hexamethylene diisocyanate, dicyclohexylmethane 4,4'-diisocyanate, isophorone diisocyanate, or a mixture thereof. In an embodiment, the diisocyanate comprises 4,4'-diphenylmethane diisocyanate (MDI), 2,4-toluene diisocyanate, 2,6-toluene diisocyanate, or 1 ,4-phenylene diisocyanate. In an embodiment, the diisocyanate consists of 4,4'-diphenylmethane diisocyanate (MDI), 2,4-toluene diisocyanate, 2,6-toluene diisocyanate, 1,4-phenylene diisocyanate, or a mixture thereof.

In an embodiment, the molecular weight of the diisocyanate is from 100 to 500 g/mol. In an embodiment, the molecular weight of the diisocyanate is from 150 to 260 g/mol.

In an embodiment, the formulation from which the polyurethane is formed comprises at least 10 wt%, at least 20 wt%, at least 25 wt%, at least 30 wt%, at least 35 wt%, or at least 40 wt% of a diisocyanate, based on the total weight of the formulation. In an embodiment, the formulation from which the polyurethane is formed comprises at most 50 wt%, at most 40 wt%, at most 35 wt%, at most 30 wt%, at most 25 wt%, or at most 20 wt% of a diisocyanate, based on the total weight of the formulation. In an embodiment, the polyurethane comprises at least 10 wt%, at least 20 wt%, at least 25 wt%, at least 30 wt%, at least 35 wt%, or at least 40 wt% of the residue of a diisocyanate, based on the polyurethane. In an embodiment, the polyurethane comprises at most 50 wt%, at most 40 wt%, at most 35 wt%, at most 30 wt%, at most 25 wt%, or at most 20 wt% of the residue of a diisocyanate, based on the total weight of the polyurethane.

### Polymeric Aliphatic Diol

The polyurethane comprises the residue of a polymeric aliphatic diol. A polymeric aliphatic diol comprises two OH groups and a backbone. The OH groups may be directly attached to the backbone or may be separated by a linker. For example, a hydroxyalkyl terminated polydimethylsiloxane (carbinol terminated) is a polymeric aliphatic diol.

The polymeric aliphatic diol comprises a polysiloxane diol and a polycarbonate diol. In an embodiment, the polymeric aliphatic diol comprises a poly(alkylene oxide), a polycarbonate, a polysiloxane, a random or block copolymer thereof, or a mixture thereof. In an embodiment, the polymeric aliphatic diol comprises a polycarbonate diol, a polysiloxane diol, a random or block polycarbonate polysiloxane copolymer diol, or a mixture thereof. In an embodiment, the polymeric aliphatic diol comprises a mixture of a polycarbonate diol and a polysiloxane diol. In an embodiment, the polymeric aliphatic diol consists of a mixture of a polycarbonate diol and a polysiloxane diol.

In an embodiment, the polymeric aliphatic diol comprises a polysiloxane diol, a polycarbonate diol, and a poly(tetramethylene oxide) diol.

In an embodiment, the polymeric aliphatic diol comprises 15 wt% or less, 10 wt% or less, 5 wt% or less, 2 wt% or less, based on the total weight of polymeric aliphatic diol, or is devoid of hydrophobic poly(alkylene oxide). Hydrophobic poly(alkylene oxide)s are polypropylene oxide) and poly(tetramethylene oxide).

In an embodiment, the polymeric aliphatic diol comprises a C₂-C₁₆ fluoroalkyl diol or C₂-C₁₆ fluoroalkyl ether diol. In an embodiment, the polyurethane backbone comprises the residue of 1H,1H,4H,4H-Perfluoro-1 ,4-butanediol, 1H,1H,5H,5H-Perfluoro-1 ,5-pentanediol, 1H,1H,6H,6H-perfluoro-1 ,6-hexanediol, 1H,1H,8H,8H-Perfluoro-1 ,8-octanediol, 1H,1H,9H,9H-Perfluoro-1,9-nonanediol, 1H,1H,10H,10H-Perfluoro-1,10-decanediol, 1H,1H,12H,12H-Perfluoro-1,12-dodecanediol, 1H,1H,8H,8H-Perfluoro-3,6-dioxaoctan-1,8-diol, 1H,1H,11H,11H-Perfluoro-3,6,9-trioxaundecan-1,11-diol. fluorinated triethylene glycol, orfluorinated tetraethylene glycol.

In an embodiment, the C₂-C₁₆ fluoroalkyl diol or C₂-C₁₆ fluoroalkyl ether diol has an Mn of at least 150 g/mol, at least 250 g/mol, or at least 500 g/mol. In an embodiment, the fluoroalkyl diol or fluoroalkyl ether diol has a Mn of at most 1500 g/mol, at most 1000 g/mol, or at most 850 g/mol. In an embodiment, the C₂-C₁₆ fluoroalkyl diol or C₂-C₁₆ fluoroalkyl ether diol is present in an amount of at least 1 wt%, at least 2 wt%, or at least 5 wt%, based on the total weight of the polyurethane. In an embodiment, the C₂-C₁₆ fluoroalkyl diol or C₂-C₁₆ fluoroalkyl ether diol is present in an amount of at most 15 wt%, at most 10 wt%, or at most 8 wt%, based on the total weight of the polyurethane.

In an embodiment, the polymeric aliphatic diol has a Mn of at least 200 g/mol, at least 250 g/mol, at least 300 g/mol, at least 400 g/mol, or at least 500 g/mol, at least 600 g/mol, at least 700 g/mol, at least 800 g/mol, at least 900 g/mol, or at least 1000 g/mol. In an embodiment, the polymeric aliphatic diol has a Mn of at most 10,000 g/mol, at most 8500 g/mol, at most 6000 g/mol, at most 5000 g/mol, at most 4000 g/mol, at most 3000 g/mol, at most 2000 g/mol, or at most 1500 g/mol.

In an embodiment, the polyurethane is formed from a formulation that comprises at least 20 wt%, at least 30 wt%, at least 40 wt%, at least 50 wt%, or at least 60 wt% of a polymeric aliphatic diol, based on the total weight of the formulation. In an embodiment, the polyurethane is formed from a formulation that comprises at most 80 wt%, at most 70 wt%, at most 60 wt%, or at most 50 wt% of a polymeric aliphatic diol, based on the total weight of the formulation. In an embodiment, the polyurethane comprises at least 20 wt%, at least 30 wt%, at least 40 wt%, at least 50 wt%, or at least 60 wt% of the residue of a polymeric aliphatic diol, based on the total weight of the polyurethane. In an embodiment, the polyurethane comprises at most 80 wt%, at most 70 wt%, at most 60 wt%, or at most 50 wt% of the residue of a polymeric aliphatic diol, based on the total weight of the polyurethane.

### Chain Extender

The polyurethane comprises the residue of a chain extender. A chain extender is an alkane diol having from 2 to 20 carbon atoms, wherein one or more carbon atoms may be substituted with oxygen. In an embodiment, the chain extender has a molecular weight of at least 60 g/mol, at least 70 g/mol, at least 80 g/mol, at least 90 g/mol, or at least 100 g/mol. In an embodiment, the chain extender has a molecular weight of at most 500 g/mol, at most from 400 g/mol, at most 300 g/mol, at most 200 g/mol, or at most 150 g/mol. In an embodiment, the chain extender comprises ethylene glycol, diethylene glycol, propylene glycol, dipropylene glycol, 1,3-propanediol, 1,4-butanediol, 1,5-pentanediol, 1,6-hexanediol, or 1,8-octanediol.

In an embodiment, the polyurethane is formed from a formulation that comprises at least 1 wt%, at least 2 wt%, at least 5 wt%, at least 8 wt%, or at least 10 wt% of a chain extender, based on the total weight of the formulation. In an embodiment, the polyurethane is formed from a formulation that comprises at most 20 wt%, at most 15 wt%, at most 12 wt%, at most 10 wt%, at most 8 wt%, or at most 5 wt%, of a chain extender, based on the total weight of the formulation. In an embodiment, the polyurethane comprises at least 1 wt%, at least 2 wt%, at least 5 wt%, at least 8 wt%, or at least 10 wt% of the residue of a chain extender, based on the total weight of the polyurethane. In an embodiment, the polyurethane comprises at most 20 wt%, at most 15 wt%, at most 12 wt%, at most 10 wt%, at most 8 wt%, or at most 5 wt%, of the residue of a chain extender, based on the total weight of the polyurethane.

### Endgroups

In an embodiment, the polyurethane comprises one or more endgroups. An endgroup is a moiety present at a terminal end of a molecule. In an embodiment, the polyurethane is linear and comprises an endgroup at each terminus of the backbone. In an embodiment, the endgroup is linear. In an embodiment, the endgroup is branched. In an embodiment, the polyurethane comprises an average of at least 0.1 endgroups, at least 0.25 endgroups, at least 0.5 endgroups, at least 1 endgroup, at least 1.5 endgroups, at least 1.8 endgroups, about 2 endgroups, or at least 2 endgroups. In an embodiment, the polyurethane comprises an average of at most 4 endgroups an average of at most 2 endgroups, or an average of at most 2 endgroups.

An endgroup may be formed by reacting a terminal isocyanate group present after forming the polymer backbone with a coreactive group on a monofunctional moiety. For instance, a terminal isocyanate group may be reacted with 1-octanol or octylamine to form a C₈ alkyl endgroup. Endgroups may also result from the inclusion of chain stoppers, such as monofunctional alcohols, in a formulation used in the formation of a polyurethane. For instance, a formulation for forming a polyurethane may comprise a diisocyanate, a polymeric aliphatic diol, a chain extender, and a monofunctional alcohol.

In an embodiment, the endgroup comprises a hydrophobic poly(alkylene oxide), a hydrophilic poly(alkylene oxide), a copolymer comprising a hydrophilic poly(alkylene oxide) and a hydrophobic poly(alkylene oxide), a polysiloxane, C₂-C₂₀ alkyl, C₂-C₁₆ fluoroalkyl, C₂-C₁₆ fluoroalkyl ether, or copolymers thereof. In an embodiment, the polysiloxane is a poly(dimethylsiloxane). In an embodiment, the hydrophilic poly(alkylene oxide) is polyethylene oxide). In an embodiment, the hydrophobic poly(alkylene oxide) is polypropylene oxide) or poly(tetramethylene oxide). In an embodiment, the endgroup comprises a hydrophobic poly(alkylene oxide), a hydrophilic poly(alkylene oxide), a copolymer comprising a hydrophilic poly(alkylene oxide) and a hydrophobic poly(alkylene oxide), C₂-C₂₀ alkyl, C₂-C₁₆ fluoroalkyl, C₂-C₁₆ fluoroalkyl ether, or copolymers thereof. Such endgroups may be formed with monofunctional alcohols, including carbinols, or amines of the foregoing.

In an embodiment, the endgroup comprises C₂-C₁₆ fluoroalkyl or C₂-C₁₆ fluoroalkyl ether. Such endgroups may be formed with monofunctional alcohols or amines comprising C₂-C₁₆ fluoroalkyl or C₂-C₁₆ fluoroalkyl ether.

In an embodiment, the endgroup is formed from a monofunctional alcohol or amine comprising C₂-C₁₆ fluoroalkyl or C₂-C₁₆ fluoroalkyl ether. In an embodiment, the endgroup is formed from 1H,1H-Perfluoro-3,6-dioxaheptan-1-ol, 1H, 1H-Nonafluoro-1-pentanol, 1H,1H-Perfluoro-1-hexyl alcohol, 1H,1H-Perfluoro-3,6,9-trioxadecan-1-ol, 1H,1H-Perfluoro-1-heptyl alcohol, 1H,1H-Perfluoro-3,6-dioxadecan-1-ol, 1H,1H-Perfluoro-1-octyl alcohol, 1H,1H-Perfluoro-1-nonyl alcohol, 1H,1H-Perfluoro-3,6,9-trioxatridecan-1-ol, 1H,1H-Perfluoro-1-decyl alcohol, 1H,1H-Perfluoro-1-undecyl alcohol, 1H,1H-Perfluoro-1-lauryl alcohol, 1H,1H-Perfluoro-1-myristyl alcohol, or 1H,1H-Perfluoro-1-palmityl alcohol.

In an embodiment, the endgroup is monomeric and has a molecular weight of 200 g/mol or more, 300 g/mol or more, or 500 g/mol or more. In an embodiment, the endgroup is monomeric and has a molecular weight of 1,000 g/mol or less or 800 g/mol or less. In an embodiment, the endgroup is polymeric and has a Mn of 10,000 g/mol or less, 8,000 g/mol or less, 6,000 g/mol or less, or 4,000 g/mol or less. In an embodiment, the endgroup is polymeric and has a Mn of 500 g/mol or more, 1,000 g/mol or more, or 2,000 g/mol or more.

In an embodiment, the endgroup is present in an amount of at least 0.1 wt%, at least 0.2 wt%, at least 0.3 wt%, or at least 0.5 wt%, based on the total weight of the formulation from which the polyurethane is formed. In an embodiment, the endgroup is present in an amount of at most 3 wt%, at most 2 wt% or at most 1 wt%, based on the total weight of the formulation from which the polyurethane is formed. In an embodiment, the endgroup is present in an amount of at least 0.1 wt%, at least 0.2 wt%, at least 0.3 wt%, or at least 0.5 wt%, based on the total weight of the polyurethane. In an embodiment, the endgroup is present in an amount of at most 3 wt%, at most 2 wt% or at most 1 wt%, based on the total weight of the polyurethane.

### Radiopacifier

The medical component comprises a radiopacifier. The radiopacifier imparts radiopacity to the medical component.

In an embodiment, the radiopacifier comprises tantalum, gold, platinum, tungsten, iridium, platinum-tungsten, platinum-iridium, palladium, rhodium, barium sulfate, bismuth subcarbonate, bismuth oxychloride, bismuth trioxide, ionic or non-ionic contrasting agents such as diatrizoates, iodipamide, iohexyl, iopamidol, iothalamate, ioversol, ioxaglate, and metrizamide, or a combination thereof. In an embodiment, the radiopacifier comprises tantalum, gold, platinum, tungsten, or a mixture or alloy thereof.

In an embodiment, the radiopacifier is present as particles. In an embodiment, the radiopacifier particles have an average particle diameter of 1 nm or more, 5 nm or more, 10 nm or more, 25 nm or more, 50 nm or more, 100 nm or more, or 200 nm or more. In an embodiment, the radiopacifier particles have an average particle diameter of 3 µm or less, 2 µm or less, 1000 nm or less, 800 nm or less, 700 nm or less, 600 nm or less, 500 nm or less, 400 nm or less, 300 nm or less, 250 nm or less, 200 nm or less, 150 nm or less 100 nm or less, or 75 nm or less. Average particle diameter is measured using photon correlation spectroscopy (PCS) in accordance with ISO13321:1996.

In an embodiment, the radiopacifier is subjected to a surface treatment with an adhesion promoter to promote adhesion to the polyurethane. In an embodiment, a composition used to form the medical component further comprises an adhesion promoter. In an embodiment, the adhesion promoter comprises a glycidyl methacrylate (GMA) modified random ethylene/acrylate copolymer, or a GMA and maleic anhydride (MA) modified random ethylene/acrylate copolymer. Commercial examples of these are Lotader^{®} AX8840, AX8900 and AX8930, produced by Arkema.

The radiopacifier is present in the medical component in an amount of 50 wt% or more, 55 wt% or more, 60 wt% or more, 65 wt% or more, 70 wt% or more, 75 wt% or more, 80 wt% or more, 85 wt% or more, 86 wt% or more, 87 wt% or more, 88 wt% or more, 89 wt% or more, or 90 wt% or more, based on the total weight of the medical component. In an embodiment, the radiopacifier is present in the medical component in an amount of 95 wt% or less, 92 wt% or less, 90 wt% or less, 88 wt% or less, 86 wt% or less, 84 wt% or less, 82 wt% or less, 80 wt% or less, or 75 wt% or less, based on the total weight of the medical component.

In an embodiment the radiopacifier is present in the composition in an amount of 50 wt% or more, based on the total dry weight of the composition. In an embodiment the radiopacifier is present in the composition in an amount of 55 wt% or less, based on the total dry weight of the composition.

### Other Optional Components

In an embodiment, the formulation for forming the polyurethane comprises a catalyst. In an embodiment, the catalyst comprises stannous octoate, dibutyltin dilaurate, or an amine catalyst.

Additional components that may be present in the medical component, the composition, or the formulation, include stabilizers, such as viscosity stabilizers, surfactants, antioxidants, or wetting agents.

In an embodiment, the medical component, the composition, or the formulation comprises a mold release agent. In an embodiment, the mold release agent is ethylene bis(stearamide).

### Solvent

The compositions used to form a medical component typically comprise a solvent. The medical component is typically formed by casting as a film a composition comprising the polyurethane, the radiopacifier, and the solvent, and evaporating the solvent. Typically, the polyurethane is first dissolved in the polyurethane, followed by dispersing the radiopacifier in the polyurethane.

In an embodiment, the solvent comprises tetrahydrofuran (THF), methyl-tetrahydrofuran (methyl-THF), dimethylacetamide (DMAc), dimethylformamide (DMF), dimethyl sulfoxide (DMSO), dichloromethane, chloroform, hexafluoroisopropanol, or a mixture thereof. In an embodiment, the solvent comprises tetrahydrofuran (THF), methyl-tetrahydrofuran (m ethyl-THF), dimethylacetamide (DMAc), dimethylformamide (DMF), dimethyl sulfoxide (DMSO), or a mixture thereof. In an embodiment, the solvent comprises tetrahydrofuran (THF) or methyl-tetrahydrofuran (methyl-THF).

A co-solvent may also be present. A co-solvent comprises less than 50 wt% of the total amount of solvent. In an embodiment, a co-solvent is present and is methanol, ethanol, isobutanol, propanol, methyl ethyl ketone, or a mixture thereof.

In an embodiment, the solvent comprises 40 wt% or more, 50 wt% or more, or 60 wt% or more of tetrahydrofuran (THF), methyl-tetrahydrofuran (methyl-THF), or a mixture thereof. In an embodiment, the solvent comprises 40 wt% or more, 50 wt% or more, or 60 wt% or more of tetrahydrofuran (THF), methyl-tetrahydrofuran (methyl-THF), or a mixture thereof, and methanol, ethanol, isobutanol, propanol, methyl ethyl ketone, or a mixture thereof at an amount of from 1 to 60 wt%, 1 to 50 wt%, or 1 to 40 wt%, based on the total amount of solvent in the composition.

The solvent is present in the composition in an amount of at least 80 wt%, at least 85 wt%, at least 88 wt%, at least 89 wt%, at least 90 wt%, at least 91 wt%, at least 92 wt%, at least 93 wt%, at least 94 wt%, or at least 95 wt%, based on the total weight of the composition. In an embodiment, the solvent is present in the composition in an amount of at most 99 wt%, at most 98 wt%, at most 97 wt%, at most 96 wt%, at most 95 wt%, or at most 94 wt%, based on the total weight of the composition.

### Formation of Medical Components

The polyurethanes may be formed as generally known in the art. A catalyst may be employed. Polyurethanes are typically provided in pellet form and should be dried prior to use.

In an embodiment, the medical component is formed from a composition. In an embodiment, the composition comprises the polyurethane, the radiopacifier, and the solvent.

A medical component may be formed from such a composition as follows. First, the polyurethane is first added to the solvent, preferably under stirring. Next, the radiopacifier is added to the composition, preferably under stirring, and dispersed. The stirring is continued until the radiopacifier is well-dispersed, taking care to avoid air bubbles.

Medical components may be formed from the composition by, for example, dip coating or solvent casting, followed by evaporating the solvent and, if necessary, separating the coating from the substrate to form the medical component. The solvent may be evaporated by merely drying in air at ambient temperature. Elevated temperatures, a vacuum, and/or a convection oven may also be used, optionally in combination with air drying. Typical elevated temperatures are from 40 to 90 °C. In an embodiment, the membrane has a residual solvent content of less than 50 ppm after drying the membrane component under nitrogen for 24 hours followed by drying in a convection oven at 50 °C for one hour.

### Medical Components

A medical component is a stand-alone article, as opposed to a coating that is constrained to a substrate. A medical component may be formed by separating a coating from a substrate such that the coating is no longer constrained to the substrate. The medical components disclosed herein can be provided in numerous forms, such as long ribbons, tapes, discs, or cylinders, such as a belt for a stent. In an embodiment, the medical component has a length of at least 5, 10, 15, 20, 25, 30, 35, 40, 45, or 50 times its thickness. In an embodiment, the medical component has a length of at most 5000, 2000, 1000, 500, 400, 300, 200, 100, 75, 50, 45, 40, 35, 30, 25, or 20 times its thickness. In an embodiment, the medical component has a length of at least 1, 2, 3, 4, 5, 6, 7, 8, 9, or 10 times its width. In an embodiment, the medical component has a length of at most 1, 2, 3, 4, 5, 6, 7, 8, 9, 10, 11, 12, 13, 14, or 15 times its width.

In an embodiment, the medical component has a length of at least 1 mm, at least 2 mm, at least 3 mm, at least 4 mm, at least 5 mm, or at least 10 mm. In an embodiment, the medical component has a length of at most 100 mm, at most 75 mm, at most 50 mm, at most 30 mm, at most 20 mm, at most 15 mm, at most 10 mm, at most 9 mm, at most 8 mm, at most 7 mm, at most 6 mm, or at most 5 mm. In an embodiment, the medical component has a width of at least 1 mm, at least 2 mm, at least 3 mm, at least 4 mm, or at least 5 mm. In an embodiment, the medical component has a width of at most 10 mm, at most 8 mm, at most 7 mm, at most 6 mm, at most 5 mm, or at most 4 mm. In an embodiment, the medical component has a length of from 2 to 10 mm and a width of from 1 to 6 mm.

In an embodiment, the medical component is a ribbon or strip. This form allows flexibility for the end user. The end user may trim the ribbon to an appropriate size and attach it to the medical device in a form that provides the desired radiopacity.

In an embodiment, the medical component is provided as a series of tabs separable from an underlying substrate. The individual tabs may be separated from the underlying substrate and attached to the medical device by the end user.

### Properties of Medical Components

In an embodiment, the medical component has a suture retention strength, as measured according to the procedure in the examples on a 29-37 mm length x 2.7-3.2 mm width x 0.17-0.2 mm thick strip, of at least 3 N, at least 3.5 N at least 4 N, at least 4.5 N, at least 5 N, at least 5.5 N, at least 6 N, at least 6.5 N, at least 7 N, at least 8 N, at least 9 N, or at least 10 N. In an embodiment, the medical component has a suture retention strength, as measured according to the procedure in the examples on a 29-37 mm length x 2.7-3.2 mm width x 0.17-0.2 mm thick strip, of at most 15 N, at most 14 N, at most 13 N, at most 12 N, at most 11 N, at most 10 N, at most 9 N, at most 8 N, at most 7 N, or at most 6 N.

In an embodiment, the medical component has a suture retention strength, as measured according to the procedure in the examples on a 29-37 mm length x 2.7-3.2 mm width x 0.17-0.21 mm thick strip, of at least 3 N, at least 3.5 N at least 4 N, at least 4.5 N, at least 5 N, at least 5.5 N, at least 6 N, at least 6.5 N, at least 7 N, at least 8N. In an embodiment, the medical component has a suture retention strength, as measured according to the procedure in the examples on a 29-37 mm length x 2.7-3.2 mm width x 0.17-0.21 mm thick strip, of at most 9 N, at most 8 N, at most 7 N, or at most 6 N.

In an embodiment, the medical component has a Young's modulus, as measured according to the procedure in the examples, of at least 40 MPa, at least 45 MPa, at least 50 MPa, at least 55 MPa, at least 56 MPa, at least 57 MPa, at least 58 MPa, at least 59 MPa, at least 60 MPa, at least 61 MPa, at least 62 MPa, at least 63 MPa, at least 64 MPa, or at least 65 MPa. In an embodiment, the medical component has a Young's modulus, as measured according to the procedure in the examples at, of at most 200 MPa, at most 175 MPa, at most 150 MPa, at most 125 MPa, at most 100 MPa, at most 75 MPa, at most 70 MPa, at most 65 MPa, at most 64 MPa, at most 63 MPa, at most 62 MPa, at most 61 MPa, or at most 60 MPa.

In an embodiment, the medical component has a Young's modulus, after submersion in phosphate buffered saline at 37 °C for one week and as measured according to the procedure in the examples, of at least 5 MPa, at least 6 MPa, at least 7 MPa, at least 8 MPa, at least 9 MPa, at least 10 MPa, at least 11 MPa, at least 12 MPa, at least 13 MPa, at least 14 MPa, or at least 15 MPa. In an embodiment, the medical component has a Young's modulus, after submersion in phosphate buffered saline at 37 °C for one week and as measured according to the procedure in the examples, of at most 50 MPa, at most 45 MPa, at most 40 MPa, at most 35 MPa, at most 30 MPa, at most 25 MPa, at most 22.5 MPa, at most 20 MPa, at most 19 MPa, at most 18 MPa, at most 17 MPa, at most 16 MPa, or at most 15 MPa.

In an embodiment, the medical component has an elongation at break, as measured according to the procedure in the examples of at least 250%, at least 275%, at least 300%, at least 325%, at least 350%, or at least 375%. In an embodiment, the medical component has an elongation at break, as measured according to the procedure in the examples of at most 450%, at most 425%, at most 400%, at most 375%, at most 370%, at most 365%, or at most 360%.

### Medical Devices

A medical component may be attached or otherwise incorporated into a medical device. In an embodiment, the medical component is secured to the medical device without the aid of an adhesive. In an embodiment, the medical component is attached to the medical device with a suture. In an embodiment, a single suture is used to secure a plurality of medical components to the medical device.

It is anticipated that the disclosed medical components may provide a benefit for medical devices that must be crimped prior to use. In an embodiment, the medical device comprises a stent, a frame for a heart valve repair or replacement, or a frame for an embolic protection device. In an embodiment, the medical device comprises a hernia mesh. In an embodiment, the medical device comprises a plurality of medical components sutured to a stent, a frame for a heart valve repair or replacement, a frame for an embolic protection device, or a hernia mesh.

In an embodiment, a medical device comprises a coating comprising the polyurethane and the radiopacifier. In an embodiment, a method for forming a medical device comprises the steps of coating a composition comprising a polyurethane, a radiopacifier, and a solvent on the body of a medical device, and evaporating the solvent, thereby forming a coating. It may be required to repeat the steps of coating and evaporating the solvent one or more times to build a sufficiently thick coating that achieves the desired radiopacity. In an embodiment, a medical device comprises a stent wherein the coating spans the struts of the stent.

The Examples below further elucidate embodiments of the invention, but of course, should not be construed as in any way limiting the scope of the claims.

### Examples

### Preparation of Test Samples

Carbosil^{®} 20-80A TSPCU has a shore hardness of 80 ShA and is a thermoplastic silicone-polycarbonate polyurethane available from DSM Biomedical BV, Sittard-Geleen NL. Carbosil^{®} 20-80A TSPCU comprises approximately 25-45 wt% of diisocyanate residue, 15-25 wt% silicone diol residue, 30-50 wt% polycarbonate diol residue, 5-15 wt% chain extender residue, and 0-3 wt% of silicone mono-ol residue.

Bionate^{®} 80A PCU has a shore hardness of 80 ShA and is a thermoplastic polycarbonate polyurethane available from DSM Biomedical BV, Sittard-Geleen NL. Bionate^{®} 80A PCU comprises approximately 20-45 wt% of diisocyanate residue, 50-75 wt% polycarbonate diol residue, and 5-15 wt% chain extender residue.

The medical components used in the examples are formed using Carbosil^{®} 80A TSPCU or Bionate^{®} 80A PCU pellets as follows. Polyurethanes typically have a moisture absorption level of 0.5-2 wt%. It is important to dry polyurethane pellets before dissolving them in solvent to obtain homogenous solutions and an improved film quality. Drying the polymer to less than 0.05% by weight moisture content is targeted. A 6.56 mass% solution of TSPCU in THF (Lichrosolve) or 12 mass% solution of PCU in THF is prepared, by first drying the polyurethane pellets overnight at 70 °C in a vacuum oven followed by the addition of THF and stirring overnight at room temperature. Once a homogenous solution is obtained, Tantalum particles (US Nanomaterials Ta Nanoparticles high purity, 99.99%, average particle size of either 50-80nm or about 500 nm, metal) are added with a fixed ratio under continuous stirring to achieve a formulation consisting of 86% w/w (30% v/v) tantalum /polyurethane, measured by dry weight.

Thin films of from 0.17-0.21 mm thickness were solvent casted using a blade coater. Multiple passes of the blade coater may be required to build up the desired thickness. Intermediate layers are air dried for 30 minutes followed by drying at 40 °C for one hour before coating the next layer.

### Measurement Methods

*Suture Retention Strength.* Test samples are cut from the films to dimensions of either 10 x 30 mm or 3 x 30 mm strips using an ultra-short pulse laser (pico-laser 800 kHz, 18W, 50 mm/sec) so as not to thermally distort the material. Commercially available sutures, USP size 4-0, composed of high-strength UHMWPE fibers (FiberWire^{®}) are inserted in the middle of the narrow side of the sample strip 2 mm from the edge of a sample using a low-profile, tapered needle. Sutures are gripped in a pneumatic clamp specifically designed for holding sutures (Instron part # 2714-040 Pneumatic Action Grips for Cord and Yarns and Instron part # 2714-044 Clamping blocks for UHMWPE type cord and yarns.) mounted on the upper position of a universal mechanical testing machine. The free end of the sample is gripped in flat clamps mounted on the lower position of the testing machine. Grip-to-grip distance is set at 200 mm at the start of each test. A pre-load of 0.05 N is applied and then the suture is tensioned at a rate of 50 mm/min until failure. The maximum tensile force at yield is recorded and denoted as the suture retention strength. The test is conducted on n=4-5 sample replicates.

*Tensile Properties.* Test samples are cut from the film to the dimensional recommendations of ISO527-2:2012, part 1BA using an ultra-short pulse laser (pico-laser 800 kHz, 18W, 50 mm/sec). The thickness of the sample is measured in the center of the sample at resting state. The test is carried out at ambient temperature and humidity, approximately 20 °C and 50% RH. Test samples are gripped on the top and bottom using flat clamps. Grip-to-grip distance is set at 62 mm at the start of each test. A pre-load of 0.05 N is applied. The sample is tensioned at a rate of 500 mm/min until failure. The maximum tensile force at yield is recorded and ultimate tensile strength is calculated. The maximum elongation before yield is recorded and denoted as elongation at break. The test is repeated on n=3-6 sample replicates.

### Example 1 - Suture Retention Strength

The suture retention strength was measured according to the above procedure. Strips of 10 x 30 mm and 3 x 30 mm were tested. The results for the 10 x 30 mm strips are shown in the following Table 1-1 and the results for the 3 x 30 mm strips are shown in the following Table 1-2. Examples designated with C are comparative examples, being without radiopacifier.

**Table 1-1 - Example 1 Results, 10 x 30 mm strips**

| Ex. | Polyurethane | Tantalum Average Particle Size | Avg. Suture Retention Strength (N) | St. Dev. (± N) |
|---|---|---|---|---|
| 1-1 | TSPCU | 50-80 nm | 5.9 | 0.4 |
| C1-2 | TSPCU | none | 6.1 | 1.4 |
| C1-3 | PCU | 50-80 nm | 7.2 | 0.5 |
| C1-4 | PCU | 500 nm | 8.2 | 0.7 |
| C1-5 | PCU | none | 8.8 | 1.6 |

**Table 1-2 - Example 2 Results, 3 x 30 mm strips**

| Ex. | Polyurethane | Tantalum Average Particle Size | Avg. Suture Retention Strength (N) | St. Dev. (± N) |
|---|---|---|---|---|
| 1-6 | TSPCU | 50-80 nm | 4.3 | 0.3 |
| C1-7 | TSPCU | none | 4.5 | 0.5 |
| C1-8 | PCU | 50-80 nm | 5.8 | 0.6 |
| C1-9 | PCU | 500 nm | 4.8 | 0.3 |
| C1-10 | PCU | none | 5.7 | 1.3 |

The examples show that the strips with radiopacifier still provide acceptable suture retention strength relative to the strips without radiopacifier. Especially the TSPCU strips show little decrease in suture retention strength when the radiopacifier is added to the polyurethane.

### Example 2 - Tensile Properties

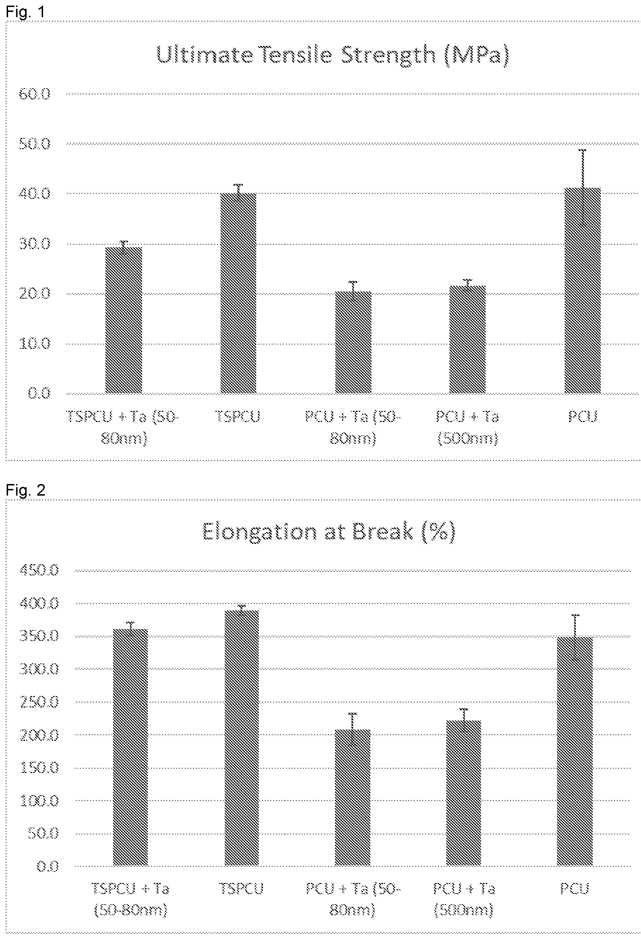

The tensile properties were measured according to the above procedure. The results for Ultimate Tensile Strength are shown in the following Table 2-1 and Fig. 1.

**Table 2-1 - Ultimate Tensile Strength**

| Ex. | Polyurethane | Tantalum Average Particle Size | Ultimate Strength (MPa) | |
|---|---|---|---|---|
| | | | Mean | Std. Dev |
| 2-1 | TSPCU | 50-80 nm | 29.3 | 1.2 |
| C2-2 | TSPCU | none | 40.1 | 1.6 |
| C2-3 | PCU | 50-80 nm | 20.5 | 1.8 |
| C2-4 | PCU | 500 nm | 21.7 | 1.0 |
| C2-5 | PCU | none | 41.2 | 7.6 |

The reduction in ultimate tensile strength of the TSPCU samples upon loading with radiopacifier is significantly less than the decrease in ultimate tensile strength of the PCU samples upon loading with radiopacifier.

The results for elongation at break are shown in the following Table 2-2 and Fig. 2.

**Table 2-2 - Elongation at Break**

| Ex. | Polyurethane | Tantalum Average Particle Size | Elongation at Break (%) | |
|---|---|---|---|---|
| | | | Mean | Std. Dev |
| 2-6 | TSPCU | 50-80 nm | 360.8 | 10.1 |
| C2-7 | TSPCU | none | 389.3 | 6.7 |
| C2-8 | PCU | 50-80 nm | 209.3 | 24.0 |
| C2-9 | PCU | 500 nm | 222.9 | 17.1 |
| C2-10 | PCU | none | 348.1 | 33.9 |

Surprisingly, the reduction in elongation at break of the TSPCU samples upon loading with radiopacifier is very small, less than 8%. Accordingly, the elongation at break of the radiopacifier-loaded TSPCU samples is surprisingly high. In contrast, the reduction in elongation at break of the PCU samples upon loading with radiopacifier is much higher (35-40%).

### Example 3 - Radiopacity

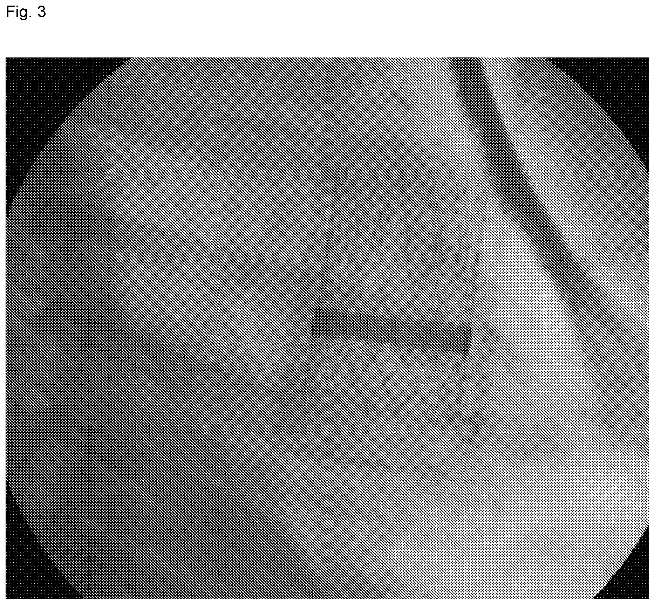

The radiopacity of the disclosed medical component was investigated in a cadaveric ex *vivo* model. A generic self-expandable nitinol stent with a diameter of 27 mm and struts of thickness 0.30 mm was used as a model cardiovascular device frame. Tantalum nanoparticles (50-80 nm) were mechanically dispersed in a Carbosil^{®} 20-80A TSPCU dissolved in tetrahydrofuran (10% w/w), equating to 86% w/w tantalum in the final solid content of the composite. The dispersion was dip-coated onto a mandrel of diameter 24 mm using a draw speed of 0.5 cm/s, air dried at ambient temperature for at least 10 minutes, then dried in an oven set at 40 °C for at least 10 minutes. This process was repeated four more times until a film thickness of 0.20 mm was achieved. The film was carefully removed from the mandrel using a scalpel and cut into rings of 4 mm width and 0.20 mm thickness. A resulting ring was fitted onto the outer surface of the stent by slightly crimping the stent and allowing it to regain its full diameter, thereby expanding the ring. The stent mounted with the composite ring was placed into a polyethylene tube to protect it during the trial. The tube containing the stent and ring was placed under the abdomen of a 50 kg sheep that had been humanely euthanized for other ethically approved research purposes. A clinical fluoroscope (ZKH7, Philips) was set to vascular abdominal mode (70 kV, 6.42 mA) and images were captured from the opposite side of the abdomen in the direction of the stent and ring underneath the cadaver. A representative image is shown in Fig. 3. The stent and radiopaque ring are clearly visible on the fluoroscope, along with the bones of the sheep.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention.

## Claims

1. A medical component comprising from 5 to 50 wt% of a polyurethane and from 50 to 95 wt% of a radiopacifier, based on the total weight of the medical component, wherein the medical component has a thickness of from 0.025 to 1 mm, and wherein the polyurethane comprises a backbone that comprises the reaction product of a diisocyanate, a polymeric aliphatic diol, and a chain extender, and wherein the polymeric aliphatic diol comprises a polysiloxane diol and a polycarbonate diol.

2. The medical component according to the previous claim, wherein the medical component has a length of from 5 to 100 mm and a width of from 1 to 6 mm.

3. The medical component according to any one of the preceding claims, wherein the polyurethane is present in an amount of from 8 to 20 wt%, the radiopacifier is present in an amount of from 80 to 92 wt%.

4. The medical component according to any one of the preceding claims, wherein the medical component has a suture retention strength, as measured according to the procedure in the examples on a 30 mm length x 3 mm width x 0.17-0.2 mm thick strip, of from 3 to 9 N.

5. The medical component according to any one of the preceding claims, wherein the medical component has an elongation at break, as measured according to the procedure in the examples, of at least 250% and at most 450%.

6. The medical component according to any one of the preceding claims, wherein the polyurethane comprises a backbone that comprises the reaction product of a diisocyanate, a polymeric aliphatic diol, and a chain extender, and comprises an endgroup comprising a polysiloxane, and wherein the polymeric aliphatic diol comprises a polysiloxane diol and a polycarbonate diol.

7. The medical component according to any one of the preceding claims, wherein the polyurethane consists of a backbone that consists of a residue of a diisocyanate, a residue of a polysiloxane diol, a residue of a polycarbonate diol, and a residue of a chain extender.

8. The medical component according to any one of the previous claims, wherein the radiopacifier comprises particles of tantalum, gold, platinum, tungsten, or a mixture or alloy thereof.

9. The medical component according to any one of the previous claims, wherein the radiopacifier is present as particles and has an average particle diameter of from 25 nm to 600 nm.

10. The medical component according to any one of the previous claims, wherein the radiopacifier is present as particles having an average particle diameter of 100 nm or less.

11. The medical component according to any one of the previous claims, wherein the medical component has a thickness of from 0.14 to 0.30 mm.

12. The medical component according to any one of the previous claims, wherein the medical component has a length of from 10 to 40 times its thickness.

13. A medical device comprising the medical component according to any one of the preceding claims.

14. The medical device according to the previous claim, wherein a plurality of the medical components, or a plurality of portions of the medical component, are attached to the medical device via a suture.

15. A method of forming a medical device comprising radiopacity comprising the steps of:
a. providing the medical component according to any one of the previous claims, and
b. attaching the medical component to a medical device via a suture.

16. A composition for forming a medical component or coating comprising:
a. from 5 to 50 wt% of a polyurethane, based on the total dry weight of the composition, wherein the polyurethane comprises a backbone that comprises the reaction product of a diisocyanate, a polymeric aliphatic diol, and a chain extender, and wherein the polymeric aliphatic diol comprises a polysiloxane diol and a polycarbonate diol,
b. 50 to 95 wt% of a radiopacifier, based on the total dry weight of the composition, and
c. from 80 to 99 wt% solvent, based on the total weight of the composition.

17. A method of forming a medical component comprising the steps of:
a. casting the composition according to the previous claim into a film,
b. evaporating the solvent, thereby obtaining the medical component,
wherein the medical component has a thickness of from 0.025 to 1 mm.

18. A medical component comprising from 8 to 20 wt% of a polyurethane and from 80 to 92 wt% of a radiopacifier, wherein the polyurethane comprises a backbone that comprises the reaction product of a diisocyanate, a polymeric aliphatic diol, and a chain extender, wherein the polymeric aliphatic diol comprises a polysiloxane diol and a polycarbonate diol, and wherein the radiopacifier is present as particles and has an average particle diameter of from 25 nm to 1000 nm.

## Patentansprüche

1. Medizinische Komponente, umfassend zu 5 bis 50 Gew.-% Polyurethan und zu 50 bis 95 Gew.-% einen Röntgenopazifizierungsmittel, bezogen auf das Gesamtgewicht of the medizinische Komponente, wobei die medizinische Komponente eine Dicke von 0,025 bis 1 mm aufweist, und wobei das Polyurethan ein Grundgerüst umfasst, das das Reaktionsprodukt eines Diisocyanats, eines polymeren aliphatischen Diols und eines Kettenverlängerers umfasst, und wobei das polymere aliphatische Diol ein Polysiloxandiol und ein Polycarbonatdiol umfasst.

2. Medizinische Komponente nach dem vorstehenden Anspruch, wobei die medizinische Komponente eine Länge von 5 bis 100 mm und eine Breite von 1 bis 6 mm aufweist.

3. Medizinische Komponente nach einem der vorstehenden Ansprüche, wobei das Polyurethan in einer Menge von 8 bis 20 Gew.-% vorliegt, das Röntgenopazifizierungsmittel in einer Menge von 80 bis 92 Gew.-% vorliegt.

4. Medizinische Komponente nach einem der vorstehenden Ansprüche, wobei die medizinische Komponente ein Nahthaltefestigkeit, gemessen gemäß dem Verfahren in den Beispielen auf einem Streifen von 30 mm Länge x 3 mm Breite x 0,17-0,2 mm Dicke, von 3 bis 9 N aufweist.

5. Medizinische Komponente nach einem der vorstehenden Ansprüche, wobei die medizinische Komponente eine Bruchdehnung, gemessen gemäß dem Verfahren in den Beispielen, von mindestens 250 % und höchstens 450 % aufweist.

6. Medizinische Komponente nach einem der vorstehenden Ansprüche, wobei das Polyurethan ein Grundgerüst umfasst, das das Reaktionsprodukt eines Diisocyanats, eines polymeren aliphatischen Diols und eines Kettenverlängerers umfasst, und umfasst eine Endgruppe, die ein Polysiloxan umfasst, und wobei das polymere aliphatische Diol ein Polysiloxandiol und ein Polycarbonatdiol umfasst.

7. Medizinische Komponente nach einem der vorstehenden Ansprüche, wobei das Polyurethan aus einem Grundgerüst besteht, das aus einem Rest eines Diisocyanats, einem Rest eines Polysiloxandiols, aus einem Rest eines Polycarbonatdiols und aus einem Rest eines Kettenverlängerers besteht.

8. Medizinische Komponente nach einem der vorstehenden Ansprüche, wobei das Röntgenopazifizierungsmittel Teilchen von Tantal, Gold, Platin, Wolfram oder einem Gemisch oder einer Legierung davon umfasst.

9. Medizinische Komponente nach einem der vorstehenden Ansprüche, wobei das Röntgenopazifizierungsmittel in Form von Teilchen vorliegt und einen durchschnittlichen Teilchendurchmesser von 25 nm bis 600 nm aufweist.

10. Medizinische Komponente nach einem der vorstehenden Ansprüche, wobei das Röntgenopazifizierungsmittel in Form von Teilchen mit einem durchschnittlichen Teilchendurchmesser von 100 nm oder weniger vorliegt.

11. Medizinische Komponente nach einem der vorstehenden Ansprüche, wobei die medizinische Komponente eine Dicke von 0,14 bis 0,30 mm aufweist.

12. Medizinische Komponente nach einem der vorstehenden Ansprüche, wobei die medizinische Komponente eine Länge des 10- bis 40-Fachen ihrer Dicke aufweist.

13. Medizinische Vorrichtung, umfassend die medizinische Komponente nach einem der vorstehenden Ansprüche.

14. Medizinische Vorrichtung nach dem vorstehenden Anspruch, wobei eine Mehrzahl der medizinischen Komponenten oder eine Mehrzahl der Abschnitte der medizinischen Komponente über eine Naht an die medizinische Vorrichtung angebracht sind.

15. Verfahren zum Bilden einer medizinischen Vorrichtung, die Röntgenopazität umfasst, umfassend die Schritte: a. Bereitstellen der medizinischen Komponente nach einem der vorstehenden Ansprüche und b. Anbringen der medizinischen Komponente an eine medizinische Vorrichtung über eine Naht.

16. Zusammensetzung zum Bilden einer medizinischen Komponente oder Beschichtung, umfassend:
a. zu 5 bis 50 Gew.-% ein Polyurethan, bezogen auf das Gesamttrockengewicht der Zusammensetzung, wobei das Polyurethan ein Grundgerüst umfasst, das das Reaktionsprodukt eines Diisocyanats, eines polymeren aliphatischen Diols und eines Kettenverlängerers umfasst, und wobei das polymere aliphatische Diol ein Polysiloxandiol und ein Polycarbonatdiol umfasst,
b. zu 50 bis 95 Gew.-% ein Röntgenopazifizierungsmittel, bezogen auf das Gesamttrockengewicht der Zusammensetzung, und
c. zu 80 bis 99 Gew.-% Lösungsmittel, bezogen auf das Gesamtgewicht der Zusammensetzung.

17. Verfahren zum Bilden einer medizinischen Komponente, umfassend die Schritte:
a. Gießen der Zusammensetzung nach dem vorstehenden Anspruch zu einer Folie,
b. Verdampfen des Lösungsmittels, wodurch die medizinische Komponente erhalten wird,
wobei die medizinische Komponente eine Dicke von 0,025 bis 1 mm aufweist.

18. Medizinische Komponente, umfassend zu 8 bis 20 Gew.-% ein Polyurethan und zu 80 bis 92 Gew.-% ein Röntgenopazifizierungsmittel, wobei das Polyurethan ein Grundgerüst umfasst, das das Reaktionsprodukt eines Diisocyanats, eines polymeren aliphatischen Diols und eines Kettenverlängerers umfasst, wobei das polymere aliphatische Diol ein Polysiloxandiol und ein Polycarbonatdiol umfasst, und wobei das Röntgenopazifizierungsmittel in Form von Teilchen vorliegt und einen durchschnittlichen Teilchendurchmesser von 25 nm bis 1000 nm aufweist.

## Revendications

1. Composant médical comprenant de 5 à 50 % en poids d'un polyuréthane et de 50 à 95 % en poids d'un radio-opacifiant, sur la base du poids total du composant médical, le composant médical ayant une épaisseur allant de 0,025 à 1 mm, et le polyuréthane comprenant un squelette qui comprend le produit de réaction d'un diisocyanate, d'un diol aliphatique polymérique, et d'un extenseur de chaîne, et le diol aliphatique polymérique comprenant un polysiloxane diol et un polycarbonate diol.

2. Composant médical selon la revendication précédente, le composant médical ayant une longueur allant de 5 à 100 mm et une largeur allant de 1 à 6 mm.

3. Composant médical selon l'une quelconque des revendications précédentes, le polyuréthane étant présent en une quantité allant de 8 à 20 % en poids, le radio-opacifiant étant présent en une quantité allant de 80 à 92 % en poids.

4. Composant médical selon l'une quelconque des revendications précédentes, le composant médical ayant une force de rétention de suture, telle que mesurée selon la procédure dans les exemples sur une bande de 30 mm de longueur x 3 mm de largeur x 0,17 à 0,2 mm d'épaisseur, allant de 3 à 9 N.

5. Composant médical selon l'une quelconque des revendications précédentes, le composant médical ayant un allongement à la rupture, comme mesuré selon la procédure dans les exemples, d'au moins 250 % et d'au plus 450 %.

6. Composant médical selon l'une quelconque des revendications précédentes, le polyuréthane comprenant un squelette qui comprend le produit de réaction d'un diisocyanate, d'un diol aliphatique polymérique, et d'un extenseur de chaîne, et comprend un groupe terminal comprenant un polysiloxane, et le diol aliphatique polymérique comprenant un polysiloxane diol et un polycarbonate diol.

7. Composant médical selon l'une quelconque des revendications précédentes, le polyuréthane étant constitué d'un squelette qui est constitué d'un radical d'un diisocyanate, d'un radical d'un polysiloxane diol, d'un radical d'un polycarbonate diol, et d'un radical d'un extenseur de chaîne.

8. Composant médical selon l'une quelconque des revendications précédentes, le radio-opacifiant comprenant des particules de tantale, d'or, de platine, de tungstène ou d'un mélange ou d'un alliage correspondant.

9. Composant médical selon l'une quelconque des revendications précédentes, le radio-opacifiant étant présent en tant que particules et possédant un diamètre moyen de particule allant de 25 nm à 600 nm.

10. Composant médical selon l'une quelconque des revendications précédentes, le radio-opacifiant étant présent en tant que particules ayant un diamètre moyen de particule de 100 nm ou moins.

11. Composant médical selon l'une quelconque des revendications précédentes, le composant médical ayant une épaisseur allant de 0,14 à 0,30 mm.

12. Composant médical selon l'une quelconque des revendications précédentes, le composant médical ayant une longueur allant de 10 à 40 fois son épaisseur.

13. Dispositif médical comprenant le composant médical selon l'une quelconque des revendications précédentes.

14. Dispositif médical selon la revendication précédente, une pluralité des composants médicaux, ou une pluralité de parties du composant médical, étant fixé(e)s au dispositif médical via une suture.

15. Procédé de formation d'un dispositif médical comprenant une radio-opacité comprenant les étapes de :
a. fourniture du composant médical selon l'une quelconque des revendications précédentes, et
b. fixation du composant médical à un dispositif médical via une suture.

16. Composition pour la formation d'un composant ou revêtement médical comprenant :
a. de 5 à 50 % en poids d'un polyuréthane, sur la base du poids total sec de la composition, le polyuréthane comprenant un squelette qui comprend le produit de réaction d'un diisocyanate, d'un diol aliphatique polymérique, et d'un extenseur de chaîne, et le diol aliphatique polymérique comprenant un polysiloxane diol et un polycarbonate diol,
b. 50 à 95 % en poids d'un radio-opacifiant, sur la base du poids total sec de la composition, et
c. de 80 à 99 % en poids de solvant, sur la base du poids total de la composition.

17. Procédé de formation d'un composant médical comprenant les étapes de :
a. coulée de la composition selon la revendication précédente en un film,
b. évaporation du solvant, obtenant ainsi le composant médical,
le composant médical ayant une épaisseur allant de 0,025 à 1 mm.

18. Composant médical comprenant de 8 à 20 % en poids d'un polyuréthane et de 80 à 92 % en poids d'un radio-opacifiant, le polyuréthane comprenant un squelette qui comprend le produit de réaction d'un diisocyanate, d'un diol aliphatique polymérique, et d'un extenseur de chaîne, le diol aliphatique polymérique comprenant un polysiloxane diol et un polycarbonate diol, et le radio-opacifiant étant présent en tant que particules et ayant un diamètre moyen de particule allant de 25 nm à 1 000 nm.
